# EUROPÄISCHE PATENTSCHRIFT

(11) **EP 0 528 375 B1**
(45) Veröffentlichungstag und Bekanntmachung des Hinweises auf die Patenterteilung: **24.04.1996**
(21) Anmeldenummer: 92113900.2
(22) Anmeldetag: 14.08.1992
(51) Int. Cl.: C07C 227/08, C07C 229/56

(54) **Verfahren zur Herstellung von 2-Amino-3-chlorbenzoesäure**
Process for the preparation of 2-amino-3-chlorobenzoic acid
Procédé pour la préparation d'acide benzoique amino-2,chloro-3

(30) Priorität: 16.08.1991 DE 4127150
(43) Veröffentlichungstag der Anmeldung: 24.02.1993
(73) Patentinhaber: HOECHST AKTIENGESELLSCHAFT, D-65926 Frankfurt am Main (DE)
(72) Erfinder: Papenfuhs, Theodor, Dr., W-6000 Frankfurt am Main 50 (DE); Rapp, Jochen, Dr., W-6000 Franfurt am Main 1 (DE)

(56) Entgegenhaltungen:
- DE-C- 244 207
- HOUBEN-WEYL, Methoden der Organischen Chemie, 4. Auflage, Band XI/1', S. 32, 33, 63-68, 1957, Georg Thieme Verlag, Stuttgart, DE
- CHEMICAL ABSTRACTS, Band 118, 1993, Columbus, Ohio, US; Zusammenfassung Nr. 233659s; & JP-A-4 356 449

## Beschreibung

Die Erfindung betrifft ein verbessertes Verfahren zur Herstellung von 2-Amino-3-chlorbenzoesäure durch Chlor-Ammoniak-Austauschreaktion (Ammonolysereaktion) von 2,3-Dichlorbenzoesäure mit wäßrigem Ammoniak in Gegenwart von kupferhaltigen Katalysatoren, wobei bei erhöhten Temperaturen und unter Druck gearbeitet wird. Das Verfahren liefert die 2-Amino-3-chlorbenzoesäure in hoher Ausbeute und sehr hoher technischer Reinheit.

2-Amino-3-chlorbenzoesäure ist ein wichtiges Zwischenprodukt zur Herstellung von pharmazeutischen Produkten und für die Landwirtschaft. 2-Amino-3-chlorbenzoesäure ist eine Vorstufe für 4-Chlor-11-oxo-11H-pyrido-[2,1-b]chinazolin-8-carbonsäure, ein Chinazolin-Derivat, das als Antiallergikum pharmazeutische Anwendung findet (DE 28 12 586 A1). Als Vorstufe für Thiazolo-[2,3-b]-chinazolone findet die 2-Amino-3-chlorbenzoesäure Anwendung in Regulatoren für das Pflanzenwachstum (DE 31 42 727 A1).

Die Herstellung von 2-Amino-3-chlorbenzoesäure erfolgte bisher beispielsweise durch Reduktion von 3-Chlor-2-nitrobenzoesäure mit Natriumdithionit in wässrigem Ammoniak (US 4347246); durch Reduktion von 3-Chlor-2-nitrobenzoesäure mit Wasserstoffgas an Platin-Katalysatoren (D. Pressman et al., Am. Soc. 76 [1954] 6336, 6337) oder durch Reduktion mit Natriumborhydrid in Methanol in Gegenwart von NiCl₂ x 6H₂O (JP 01299259). Nachteilig an den vorstehend beschriebenen Darstellungsmethoden ist, daß allen die recht schwer zugängliche 3-Chlor-2-nitrobenzoesäure zugrunde liegt.

Aus der DE-C 244 207 geht ein Verfahren zur Herstellung von Chlorsubstitutionsprodukten der Anthranilsäure durch Umsetzung von Polychlorsäuren, die sich von der O-Chlorbenzoesäure ableiten, mit Ammoniak in Gegenwart von Kupfer oder Kupferverbindungen hervor. Es wird ausdrücklich empfohlen, unter nicht allzu energischen Bedingungen, d.h. bei Temperaturen von lediglich 100 bis 150°C zu arbeiten. 2,3-Dichlorbenzoesäure wird als Polychlorsäure namentlich nicht erwähnt. Das Verfahren wird durch zwei Beispiele, nämlich durch die Umsetzung von 2,4-Dichlorbenzoesäure bei 120°C und von 2,4,5-Trichlorbenzoesäure bei 135 bis 140°C belegt.

Eine weitere Darstellungsmethode geht von 7-Chlor-indolin-2,3-dion aus, dessen Umsetzung mit Wasserstoffperoxyd-Lösung in wäßriger Natronlauge zur 3-Chloranthranilsäure führt (B.R. Baker et al., J. org. Chem. 17 [1952] 141,143; P.W. Sadler, R.L. Warren, Am. Soc. 78 [1956] 1251, 1254). Besonders nachteilig an diesem Syntheseweg ist, daß die Kondensation von o-Chloranilin, Chloral und Hydroxylamin das 7-Chlor-indolin-2,3-dion (7-Chlorisatin) nur in einer Ausbeute von 35% d. Th. ergibt und die Hydrolyse des 7-Chlorisatins die 3-Chloranthranilsäure nur in einer Ausbeute von 78% d. Th. (27,3% Gesamt-Ausbeute über beide Stufen) liefert. Ein alternatives Verfahren, der Hoffmannabbau der 3-Chlorphthalaminsäure, ergibt 47% d. Th. an 2-Amino-3-chlorbenzoesäure (G.S. Patel et al., J. Indian Chem. Soc. 34 [1957] 373).

Die geringen Ausbeuten der oben beispielsweise genannten bekannten Verfahren als auch die nicht vorhandene Verfügbarkeit der schwer zugänglichen Ausgangsverbindungen machen eine technische Herstellung unter ökologischen und ökonomischen Gesichtspunkten unmöglich.

Es bestand somit ein Bedarf für ein verbessertes, technisches Verfahren zur Herstellung von 2-Amino-3-chlorbenzoesäure, das unter Verwendung von leicht zugänglichen und technisch verfügbaren Ausgangsverbindungen in einer Verfahrensstufe ein in hoher Reinheit und hohen Ausbeuten und möglichst durch einfache Fittration isolierbares Produkt ergibt.

Es wurde nun überraschenderweise gefunden, daß man 2-Amino-3-chlorbenzoesäure in hoher Ausbeute und sehr hoher technischer Reinheit herstellen kann, indem man 1 Mol 2,3-Dichlorbenzoesäure mit der mindestens äquimolaren Menge eines Alkalimetallhydroxids in 400 bis 2000 Teilen Wasser löst und anschließend mit 500 bis 2500 mol-% Ammoniak bei Temperaturen von 150° bis 220°C, vorzugsweise von 160° bis 190°C, besonders bevorzugt von 165° bis 175°C umsetzt.

Verwendet man eine wäßrige Ammoniaklösung, so kann man beispielsweise eine handelsübliche 25%-ige Lösung einsetzen. In eine solche Lösung kann jedoch, falls gewünscht, eine zusätzliche Menge Ammoniak eingebracht werden, wobei die obere Grenze bei einer etwa 60%-igen wäßrigen Lösung liegt. Die Anwendung einer wäßrigen Ammoniaklösung, deren Ammoniakgehalt wesentlich unter 25% liegt, empfiehlt sich nicht, weil damit eine unerwünschte Verlängerung der Reaktionszeit und eine erniedrigte Raum-Zeit-Ausbeute verbunden wäre.

Als Alkalimetallhydroxide kommen Lithium-, Natrium-, Kalium-, Rubidium-oder Cäsiumhydroxid in Frage. Bevorzugt wird Kaliumhydroxid, besonders bevorzugt Natriumhydroxid verwendet. Die Alkalimetallhydroxide können in fester Form oder in Form einer wäßrigen Lösung eingesetzt werden. Setzt man Lösungen der Alkalimetallhydroxide ein, so wird die Wassermenge des Verfahrens entsprechend reduziert.

Beim erfindungsgemäßen Verfahren wird das primär gebildete 2,3-Dichlorbenzoat (1 Mol) zweckmäßigerweise in 400 bis 2000 Teilen Wasser, vorzugsweise in 450 bis 1500 Teilen Wasser, besonders bevorzugt in 500 bis 600 Teilen Wasser mit 500 bis 2500 mol-% Ammoniak, vorzugsweise mit 780 bis 1550 mol-% Ammoniak, besonders bevorzugt mit 1200 bis 1300 mol-% Ammoniak umgesetzt, wobei das 2,3-Dichlorbenzoat bei Verfahrensbeginn im Wasser gelöst oder suspendiert vorliegen kann. Das Ammoniak kann flüssig, gasförmig oder in Form konzentrierter wäßriger Lösungen zugesetzt werden. Im Falle der Anwendung konzentrierter wäßriger Ammoniaklösungen ist die Wassermenge entsprechend zu reduzieren.

Als Kupferkatalysatoren können Kupfer-Bronze, Kupfer (I)- und/oder Kupfer (II)-Salze, wie beispielsweise die Halogenide, wie beispielsweise die Chloride, oder die Oxide, Nitrate, Sulfate, Carbonate oder Acetate der 1- oder 2-wertigen Kupfers oder deren Gemische verwendet werden. Bevorzugt wird das Kupfer (I)-chlorid verwendet. Die Kupferkatalysatoren werden zweckmäßigerweise in einer Menge von etwa 20 hol-%, vorzugsweise von etwa 5 mol-%, bezogen auf die 2,3-Dichlorbenzoesäure, eingesetzt. Bevorzugt wird Kupfer (I)-chlorid in einem Mengenverhältnis von etwa 2 bis etwa 20 mol-%, besonders bevorzugt von etwa 1,8 bis etwa 5 mol-% eingesetzt.

Bei den weiter oben genannten Bereichen der Reaktionstemperaturen stellt sich ein Druck von etwa 20 bis etwa 60 bar ein, weshalb die Reaktion zweckmäßigerweise bzw. zwingend im Autoklav durchgeführt wird.

Die Umsetzung benötigt zwischen 30 Minuten und 10 Stunden, normalerweise zwischen 1 und 3 Stunden. Nach beendeter Reaktion wird das überschüssige Ammoniak durch Destillation zurückgewonnen und rückgeführt. Durch Sauerstellung des Sumpfes mit konzentrierter Salzsäure oder Schwefelsäure auf pH 3-4 wird das Produkt ausgefällt. Anschließend wird es gesammelt und mit Wasser gewaschen.

Die nachstehenden Beispiele dienen zur Erläuterung des Verfahrens, ohne es darauf zu beschränken. Teile bedeuten Gewichtsteile.

### Beispiel 1

In einem 2l V4A Autoklav werden 191 Teile 2,3-Dichlorbenzoesäure, 80 Teile 50%-ige wäßrige Natronlauge, 1004 Teile 25%-ige wäßrige NH₃-Lösung und 5 Teile CuCl vorgelegt. Es werden noch 89 Teile NH₃ flüssig (d₂₀ : 0,61) eingedrückt und der Autoklav 5 Stunden auf 170°C erhitzt, wobei der Druck von anfänglich 25 bar auf ca. 22 bar abfällt. Nach beendeter Reaktion wird der Autoklavinhalt auf 25°C abgekühlt und der Autoklav über die Steigleitung entleert. Die Reaktionslösung wird, bis 100°C Sumpftemperatur erreicht sind, am Rückfluß gekocht und das abgetriebene NH₃-Gas in einem Abgaswäscher aufgefangen. Nach Kühlung auf 25°C wird die Reaktionslösung mit 122 Teilen 30%-iger Salzsäure auf pH 3 gestellt. Die ausgefallene 2-Amino-3-chlorbenzoesäure wird abgesaugt, mit 300 Teilen Wasser gewaschen und bei 80°C im Vakuum getrocknet. Es werden 145 Teile (85% d. Th.) 2-Amino-3-chlorbenzoesäure mit einem Fp von 185° bis 187°C erhalten.

Verwendet man anstelle von 5 Teilen CuCl 10 Teile Cu(OH)₂·2H₂O oder 8 Teile CuCl₂·2H₂O und verfährt im übrigen wie vorstehend beschrieben, so gelangt man praktisch zum gleichen Ergebnis.

### Beispiel 2

In einem 2l V4A Autoklav werden eine Lösung, bestehend aus 191 Teilen 2,3-Dichlorbenzoesäure und 40 Teilen NaOH Prills in 500 Teilen Wasser, und 3 Teile CuCl vorgelegt. Anschließend werden noch 425 Teile NH₃, flüssig (d₂₀ : 0,61) eingedrückt und der Autoklav 3 Stunden auf 165° bis 175°C erhitzt, wobei der Druck von anfänglich 50 bar auf ca. 46 bar abfällt. Nach beendeter Reaktion wird der Autoklavinhalt auf 25°C abgekühlt und über die Steigleitung entleert. Die Reaktionslösung wird, bis 100°C Sumpftemperatur erreicht sind, am Rückfluß gekocht und das abgetriebene NH₃-Gas in einem Abgaswäscher aufgefangen. Aufarbeitung gemäß Beispiel 1 ergibt 168 Teile (98% d. Th.) 2-Amino-3-chlorbenzoesäure mit einem Fp von 185° bis 187°C.

Verwendet man anstelle von 3 Teilen CuCl 6 Teile Kupfer-Bronze oder 5 Teile CuCl₂·2H₂O und verfährt man im übrigen wie vorstehend beschrieben, so gelangt man praktisch zum gleichen Ergebnis.

## Patentansprüche

1. Verfahren zur Herstellung von 2-Amino-3-chlorbenzoesäure, dadurch gekennzeichnet, daß man 1 Mol 2,3-Dichlorbenzoesäure mit der mindestens äquimolaren Menge eines Alkalimetallhydroxids oder mit der wäßrigen Lösung der äquimolaren Menge eines Alkalimetallhydroxids in 400 bis 2000 Teilen Wasser löst und anschließend mit 500 bis 2500 mol-% Ammoniak bei Temperaturen von 150° bis 220°C in Gegenwart von Kupfer-Bronze, Kupfer (I)- und/oder Kupfer (II)-salzen umsetzt.

2. Verfahren nach Anspruch 1, dadurch gekennzeichnet, daß man bei Temperaturen von 160° bis 190°C umsetzt.

3. Verfahren nach Anspruch 1, dadurch gekennzeichnet, daß man bei Temperaturen von 165° bis 175°C umsetzt.

4. Verfahren nach mindestens einem der Ansprüche 1 bis 3, dadurch gekennzeichnet, daß man in Gegenwart von Halogeniden, Oxiden, Nitraten, Sulfaten, Carbonaten oder Acetaten des 1- oder 2-wertigen Kupfers oder deren Gemischen als Katalysatoren umsetzt.

5. Verfahren nach mindestens einem der Ansprüche 1 bis 4, dadurch gekennzeichnet, daß man in Gegenwart von Kupfer (I)-chlorid als Katalysator umsetzt.

6. Verfahren nach mindestens einem der Ansprüche 1 bis 5, dadurch gekennzeichnet, daß man mit 780 bis 1550 mol-% Ammoniak umsetzt.

7. Verfahren nach mindestens einem der Ansprüche 1 bis 6, dadurch gekennzeichnet, daß man mit 1200 bis 1300 mol-% Ammoniak umsetzt.

8. Verfahren nach mindestens einem der Ansprüche 1 bis 7, dadurch gekennzeichnet, daß man als Alkalimetallhydroxid Natriumhydroxid oder Kaliumhydroxid verwendet.

9. Verfahren nach mindestens einem der Ansprüche 1 bis 8, dadurch gekennzeichnet, daß man die 2,3-Dichlorbenzoesäure mit dem Alkalimetallhydroxid in 450 bis 1500 Teilen Wasser löst.

10. Verfahren nach mindestens einem der Ansprüche 1 bis 8, dadurch gekennzeichnet, daß man die 2,3-Dichlorbenzoesäure mit dem Alkalimetallhydroxid in 500 bis 600 Teilen Wasser löst.

11. Verfahren nach mindestens einem der Ansprüche 1 bis 10, dadurch gekennzeichnet, daß man das Ammoniak flüssig, gasförmig oder in Form konzentrierter wäßriger Lösungen anwendet.

12. Verfahren nach mindestens einem der Ansprüche 1 bis 11, dadurch gekennzeichnet, daß man nach beendeter Umsetzung das überschüssige Ammoniak destillativ zurückgewinnt und für einen neuen Ansatz verwendet.

## Claims

1. A process for the preparation of 2-amino-3-chlorobenzoic acid, which comprises dissolving 1 mol of 2,3-dichlorobenzoic acid in 400 to 2000 parts of water with at least the equimolar amount of an alkali metal hydroxide or with the aqueous solution of the equimolar amount of an alkali metal hydroxide, and subsequently reacting it with 500 to 2500 mol % of ammonia at temperatures from 150° to 220°C in the presence of copper bronze, copper(I) salts and/or copper(II) salts.

2. The process as claimed in claim 1, wherein the reaction is carried out at temperatures from 160° to 190°C.

3. The process as claimed in claim 1, wherein the reaction is carried out at temperatures from 165° to 175°C.

4. The process as claimed in at least one of claims 1 to 3, wherein the reaction is carried out in the presence of halides, oxides, nitrates, sulfates, carbonates or acetates of 1- or 2-valent copper or mixtures thereof as catalysts.

5. The process as claimed in at least one of claims 1 to 4, wherein the reaction is carried out in the presence of copper(I) chloride as the catalyst.

6. The process as claimed in at least one of claims 1 to 5, wherein the reaction is carried out with 780 to 1550 mol % of ammonia.

7. The process as claimed in at least one of claims 1 to 6, wherein the reaction is carried out with 1200 to 1300 mol % of ammonia.

8. The process as claimed in at least one of claims 1 to 7, wherein sodium hydroxide or potassium hydroxide is used as the alkali metal hydroxide.

9. The process as claimed in at least one of claims 1 to 8, wherein the 2,3-dichlorobenzoic acid is dissolved in 450 to 1500 parts of water with the alkali metal hydroxide.

10. The process as claimed in at least one of claims 1 to 8, wherein the 2,3-dichlorobenzoic acid is dissolved in 500 to 600 parts of water with the alkali metal hydroxide.

11. The process as claimed in at least one of claims 1 to 10, wherein the ammonia is used in the liquid or gaseous form or in the form of concentrated aqueous solutions.

12. The process as claimed in at least one of claims 1 to 11, wherein, when the reaction has ended, the excess ammonia is recovered by distillation and is used for a new batch.

## Revendications

1. Procédé pour la préparation d'acide 2-amino-3-chlorobenzoïque caractérisé en ce qu'on dissout 1 mole d'acide 2,3-dichlorobenzoïque avec la quantité au moins équimolaire d'un hydroxyde de métal alcalin ou avec la solution aqueuse de la quantité équimolaire d'un hydroxyde de métal alcalin dans 400 à 2 000 parties d'eau et ensuite on fait réagir avec 500 à 2 500 % en moles d'ammoniac à des températures de 150 à 220 °C en présence de bronze au cuivre, de sel de cuivre(I) et/ou cuivre(II).

2. Procédé selon la revendication 1, caractérisé en ce qu'on effectue la réaction à des températures de 160 à 190 °C.

3. Procédé selon la revendication 1, caractérisé en ce qu'on effectue la réaction à des températures de 165 à 175 °C.

4. Procédé selon au moins l' une des revendications 1 à 3, caractérisé en ce qu'on effectue la réaction en présence d' halogénures, d'oxydes, de nitrates, de sulfates, de carbonates ou d'acétates de cuivre mono- ou bivalent ou de leurs mélanges, en tant que catalyseurs.

5. Procédé selon au moins l'une des revendications 1 à 4, caractérisé en ce qu'on effectue la réaction en présence de chlorures de cuivre(I) en tant que catalyseurs.

6. Procédé selon au moins l' une des revendications 1 à 5, caractérisé en ce qu'on effectue la réaction avec 780 à 1 550 % en moles d'ammoniac.

7. Procédé selon au moins l'une des revendications 1 à 6, caractérisé en ce qu'on effectue la réaction avec 1200 à 1300 % en moles d'ammoniac.

8. Procédé selon au moins l'une des revendications 1 à 7, caractérisé en ce qu'on utilise en tant qu'hydroxyde de métal alcalin l'hydroxyde de sodium ou l'hydroxyde de potasslum.

9. Procédé selon au moins l'une des revendications 1 à 8, caractérisé en ce qu'on dissout l'acide 2,3-dichlorobenzoïque avec l'hydroxyde de métal alcalin dans 450 à 1 500 parties d'eau.

10. Procédé selon au moins l'une des revendications 1 à 8, caractérisé en ce qu'on dissout l'acide 2,3-dichlorobenzoïque avec l'hydroxyde de métal alcalin dans 500 à 600 parties d'eau.

11. Procédé selon au moins l'une des revendications 1 à 10, caractérisé en ce qu'on utilise l'ammoniac liquide, gazeux ou sous forme de solution aqueuse concentrée.

12. Procédé selon au moins l'une des revendications 1 à 11, caractérisé en ce qu'après avoir terminé la réaction on récupère l'excès en ammoniac par distillation et on le réutilise dans la nouvelle charge.
